# EUROPEAN PATENT APPLICATION

(11) **EP 4 799 682 A1**
(43) Date of publication of application: **02.09.2026**
(21) Application number: 26156005.6
(22) Date of filing: 03.02.2026
(51) Int. Cl.: A61N 5/10, G21K 1/04

(54) **MULTI-LEAF COLLIMATORS AND RADIOTHERAPY MACHINES INCLUDING THE SAME**

(30) Priority: 28.02.2025 US 202519067288
(71) Applicant: Varian Medical Systems, Inc., Palo Alto, CA 94304 (US)
(72) Inventor: SCOLLAY, Stuart, 94304 Palo Alto, CA (US)
(74) Representative: HKW Intellectual Property PartG mbB

(57) **Abstract**

A multilayer multi-leaf collimator (MLC) includes outboard shielding components that span or extend across the treatment field from a guide feature in a first leaf box to a matching guide feature in an opposing second leaf box. Unlike pairs of beam blocking leaves, which extend to a central portion of a radiation therapy field, each of the outboard shielding components extends from within an outer edge of one leaf box across the treatment field to within a corresponding outer edge of the opposing leaf box.

## Description

### TECHNICAL FIELD

One or more example embodiments relate to multi-leaf collimators (MLCs) and radiotherapy treatment delivery machines including the same.

### BACKGROUND

Multi-leaf collimators (MLCs) are used in radiotherapy treatment delivery machines to support radiation therapy treatments such as intensity-modulated radiation therapy (IMRT) and others. Conventional MLCs include sets of beam blocking leaves arranged in two opposing banks. In operation, each of the individual beam blocking leaves is positioned to block a portion of a radiation beam passing through the volume occupied by the leaf. The combined positioning of all beam blocking leaves defines one or many apertures through which the unblocked radiation beam passes, and the aperture(s) define(s) the shape of the radiation beam directed to a treatment field at an isocenter.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

At least one example embodiment provides a multi-leaf collimator to define a treatment field for a radiation therapy treatment machine, the multi-leaf collimator comprising: a first leaf box configured to move in a first direction and configured to hold a plurality of first beam blocking leaves arranged in a first row, wherein the plurality of first beam blocking leaves are configured to move relative to one another and relative to the first leaf box; a second leaf box arranged opposite the first leaf box, the second leaf box configured to move in the first direction and configured to hold a plurality of second beam blocking leaves arranged in a second row, wherein the plurality of second beam blocking leaves are configured to move relative to one another, configured to move relative to the plurality of first beam blocking leaves, and configured to move relative to the second leaf box; and at least a first edge defining leaf extending between the first leaf box and the second leaf box, the first edge defining leaf arranged at a first end of the first row and at a first end of the second row, wherein the first edge defining leaf is fixed relative to the first leaf box and configured to move relative to the second leaf box.

At least one other example embodiment provides a radiation therapy treatment machine comprising: a radiation source configured to emit a radiation beam; and a multi-leaf collimator to block portions of the radiation beam to define a treatment field for the radiation therapy treatment machine, the multi-leaf collimator including a first leaf box configured to move in a first direction and configured to hold a plurality of first beam blocking leaves arranged in a first row, wherein the plurality of first beam blocking leaves are configured to move relative to one another and relative to the first leaf box, a second leaf box arranged opposite the first leaf box, the second leaf box configured to move in the first direction and configured to hold a plurality of second beam blocking leaves arranged in a second row, wherein the plurality of second beam blocking leaves are configured to move relative to one another, configured to move relative to the plurality of first beam blocking leaves, and configured to move relative to the second leaf box, and at least a first edge defining leaf extending between the first leaf box and the second leaf box, the first edge defining leaf arranged at a first end of the first row and at a first end of the second row, wherein the first edge defining leaf is fixed relative to the first leaf box and configured to move relative to the second leaf box.

According to one or more example embodiments, the first edge defining leaf may define an edge of the treatment field.

The multi-leaf collimator may include: a second edge defining leaf extending between the first leaf box and the second leaf box, the second edge defining leaf arranged at a second end of the first row and a second end of the second row, wherein the second edge defining leaf is fixed relative to the second leaf box and configured to move relative to the first leaf box.

The first leaf box may be configured to hold a plurality of third beam blocking leaves arranged in a third row below the plurality of first beam blocking leaves, wherein the plurality of third beam blocking leaves may be configured to move relative to one another and relative to the first leaf box. The second leaf box may be configured to hold a plurality of fourth beam blocking leaves arranged in a fourth row below the plurality of second beam blocking leaves, wherein the plurality of fourth beam blocking leaves may be configured to move relative to one another, configured to move relative to the plurality of third beam blocking leaves, and configured to move relative to the second leaf box. The multi-leaf collimator may further include a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

The multi-leaf collimator may further include a second scatter-blocking leaf extending between the first leaf box and the second leaf box, the second scatter-blocking leaf arranged below the second edge defining leaf at a second end of the third row and a second end of the fourth row, wherein the second scatter-blocking leaf is fixed relative to the second leaf box and configured to move relative to the first leaf box.

The first edge defining leaf and the second edge defining leaf may define edges of the treatment field.

The first leaf box may be configured to move the first edge defining leaf relative to the second leaf box, and the second leaf box may be configured to move the second edge defining leaf relative to the first leaf box.

The first edge defining leaf, the plurality of first beam blocking leaves, and the plurality of second beam blocking leaves may be configured to move relative to one another.

The first leaf box may be configured to move the first edge defining leaf relative to the second leaf box, and the second leaf box may include a guide component to hold the first edge defining leaf at an edge of the treatment field as the first edge defining leaf moves relative to the second leaf box.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIG. 1 is a side perspective view of a multi-leaf collimator (MLC) according to example embodiments.
FIG. 2A and FIG. 2B are front perspective views of the MLC shown in FIG. 1
FIG. 3 is a side view of the MLC shown in FIG. 1.
FIG. 4 illustrates the MLC shown in FIG. 1 with the leaf boxes removed.
FIG. 5 and FIG. 6 are perspective views of the shielding elements and retaining mechanisms of the MLC shown in FIG. 1.
FIG. 7 illustrates a perspective view of a leaf box and the portion of the MLC shown in FIG. 5 and FIG. 6.
FIG. 8 is a perspective view of leaf boxes and outboard shielding elements of the MLC shown in FIG. 1.
FIG. 9 illustrates a proximal and distal outboard shielding element according to example embodiments.
FIG. 10 is a perspective view of a leaf box according to example embodiments.
FIG. 11 illustrates another view of the leaf box shown in FIG. 10.
FIG. 12 illustrates the leaf box shown in FIGS. 10 and 11 including shielding elements according to example embodiments.
FIG. 13 is a top view of a MLC according to other example embodiments.
FIG. 14 illustrates an example of a radiation therapy treatment machine, according to example embodiments;
FIG. 15 is a block diagram illustrating an example embodiment of the controller 220 shown in FIG. 1.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

While operations in one or more figures may be presented as occurring in series and in a certain order, example embodiments are not so limited. The operations may be performed in a different order and/or in parallel, and they may also be performed in an iterative manner.

Conventional radiotherapy devices include a primary collimation system upstream from a multi-leaf collimator (MLC). The primary collimator is a fixed aperture that initially shapes the radiation beam emerging from the linear accelerator, providing a basic level of beam shaping and limiting the maximum field size.

Downstream of the primary collimator, movable x and y jaws provide further refinement of the beam shape. These jaws may be adjusted independently to create a rectangular or square field of varying dimensions.

With dual-layer stacked MLCs, jaws may be omitted to save vertical space in the treatment head. However, with the reduction in pre-collimation prior to the MLC, there is the potential for field leakage to the side of the leaf banks. Additional outboard or side shielding leaves or components are used to mitigate and/or prevent this field leakage.

Additionally, in a conventional stacked MLC with movable leaf boxes, accurately placing shielding components may be relatively difficult since the outboard shielding components must clear the leaf boxes as the leaf boxes move.

One or more example embodiments provide multilayer MLCs including outboard shielding components (e.g., edge defining and/or scatter-blocking leaves) that span or extend across a radiation therapy treatment field from a guide feature (also referred to as a guide groove or guide component) in one leaf box to a matching guide feature in an opposing leaf box. Unlike pairs of beam blocking leaves, which moveably extend to a central portion of a radiation therapy treatment field, each of the outboard shielding components extends from within an outer edge of one leaf box across the treatment field to within a corresponding outer edge of the opposing leaf box.

By spanning between the two leaf boxes, the placement of the outboard shielding components may have the same or substantially the same tolerances as the other beam blocking leaves of the MLC.

Outboard shielding components may include edge defining leaves and/or scatter-blocking leaves. The edge defining leaves define and shape the opposing edges of a radiation therapy treatment field for a patient during treatment. The scatter-blocking leaves block or reduce the scattered radiation that reaches healthy tissue of the patient. Outboard shielding components may also be referred to as outboard shielding elements, boundary shielding elements or boundary shielding components.

As described herein, beam blocking leaves, edge defining leaves and/or scatter-blocking leaves may be referred to more generally as shielding elements or components. Shielding elements may be formed of one or more high density materials, such as tungsten.

MLCs, according to one or more example embodiments, are designed such that a single leaf box may be removed during a service event by retracting the leaf boxes to their maximum limits, removing the retaining mechanism and sliding the shielding component deeper into the leaf box such that the shielding component slides out of the opposite leaf box. In at least this regard, the outboard shielding component(s) need not always span both leaf boxes and do(es) not require both leaf boxes to be removed together.

According to one or more example embodiments, each outboard shielding component may be fixed or stationary relative to a first leaf box and configured to move relative to the opposing second leaf box. In one example, the proximal and distal outboard shielding components (e.g., the edge defining leaf and scatter-blocking leaf) at one side of the treatment field (or MLC) may move along with the first leaf box, but move relative to a second opposing leaf box, whereas the proximal and distal outboard shielding components at the opposite side of the treatment field (or MLC) may move along with the second leaf box, but move relative to the first leaf box.

According to at least one other example embodiment, each outboard shielding component may be fixed in position by a retaining bracket at a middle portion of the respective outboard shielding element. In this example embodiment, each leaf box moves relative to the outboard shielding elements.

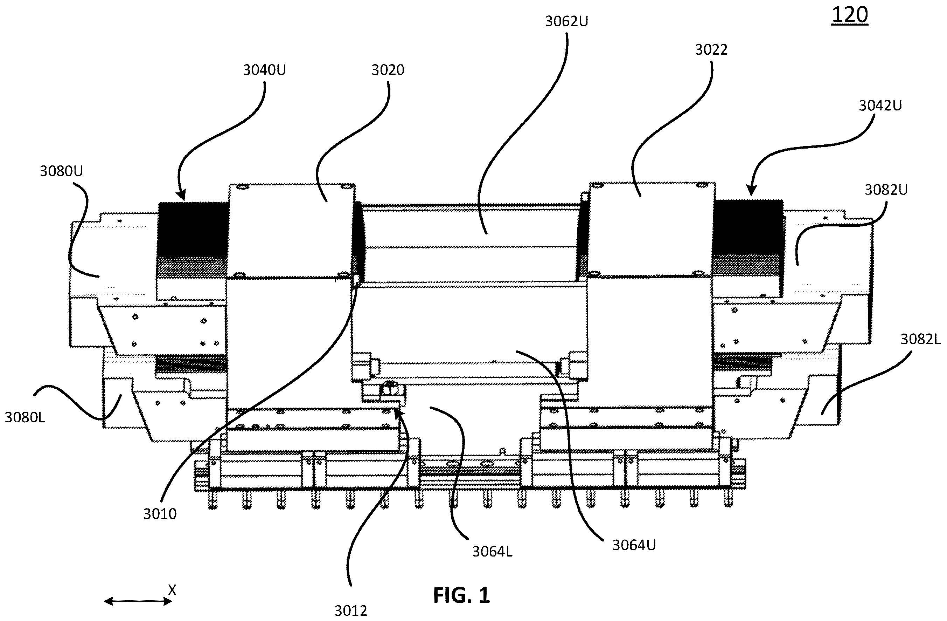

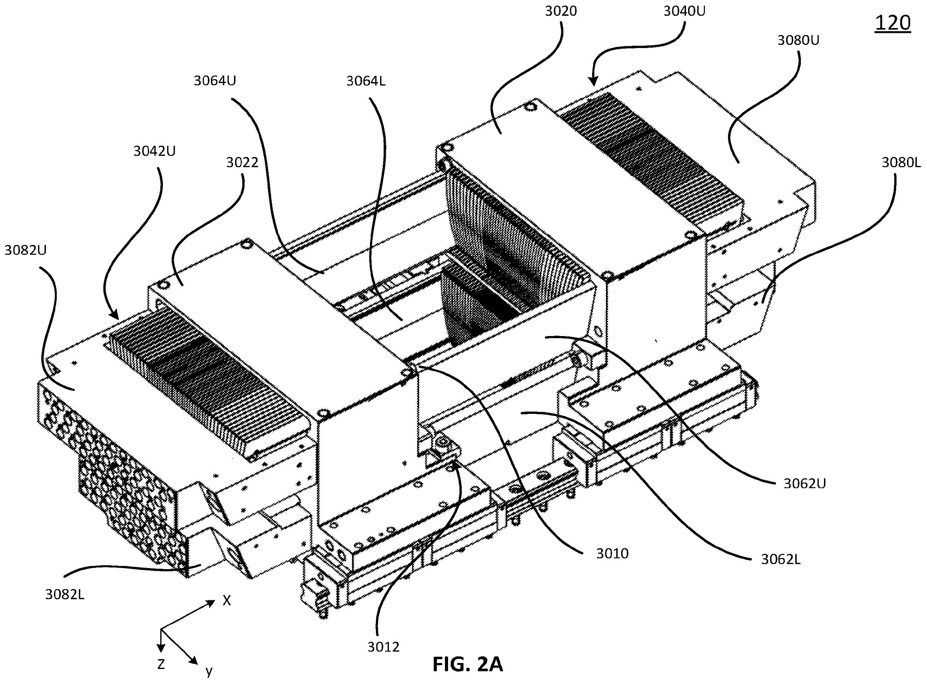

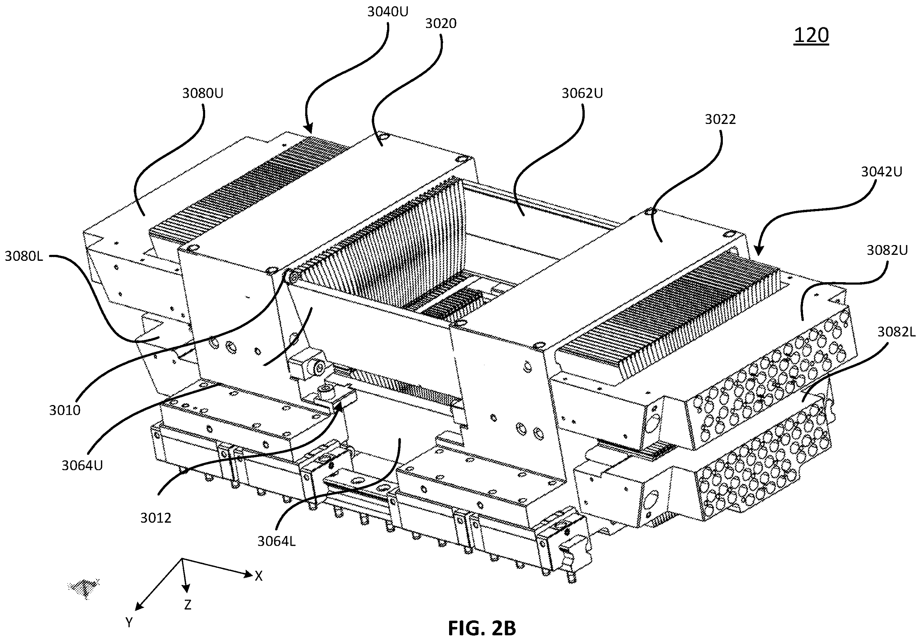

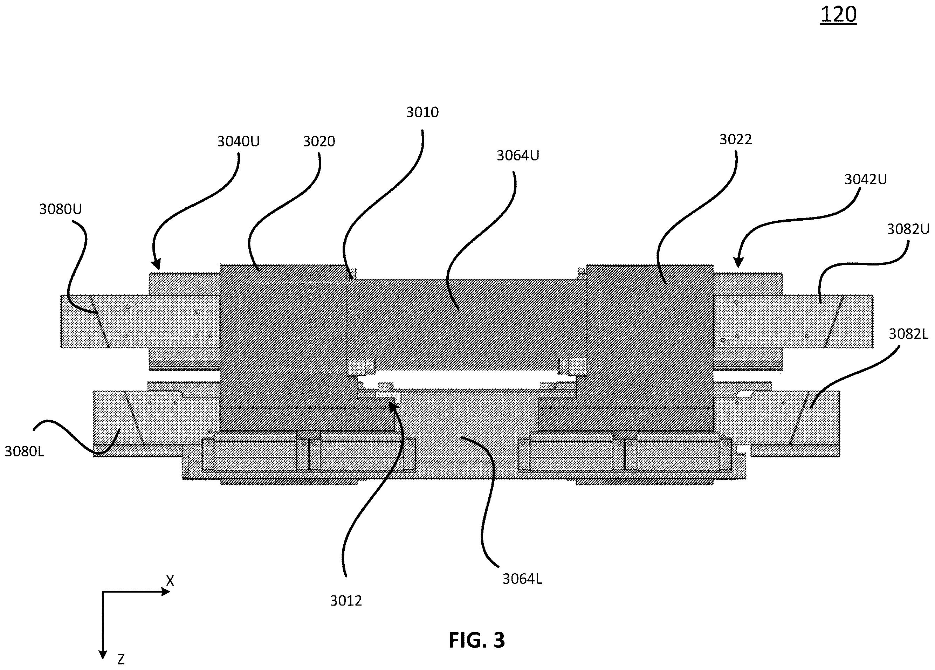

Example embodiments of MLCs, and components thereof, will now be described in detail with regard to FIGS. 1-13.

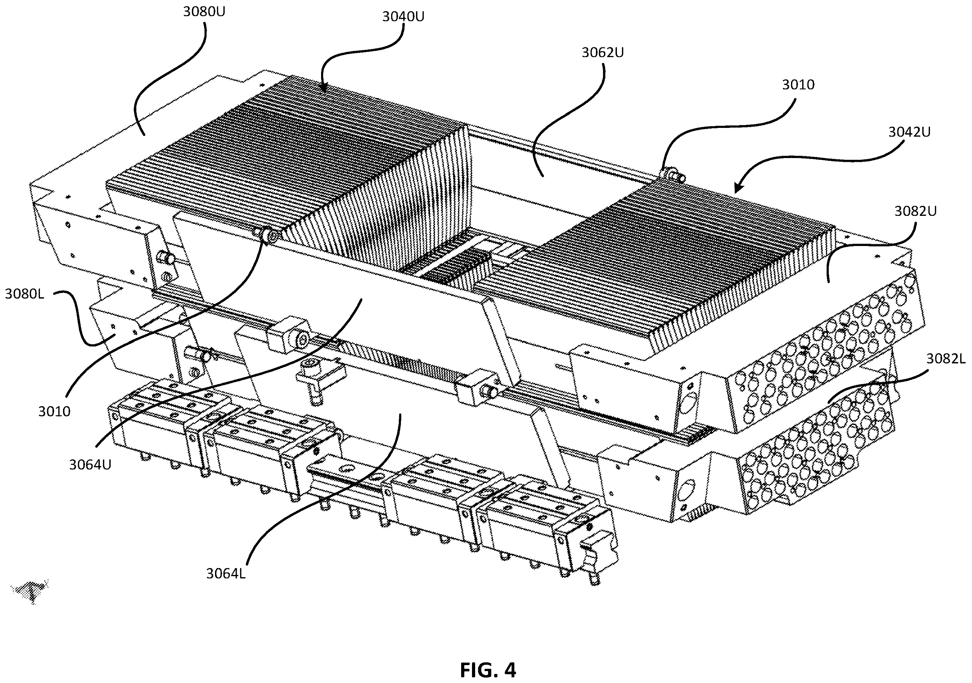

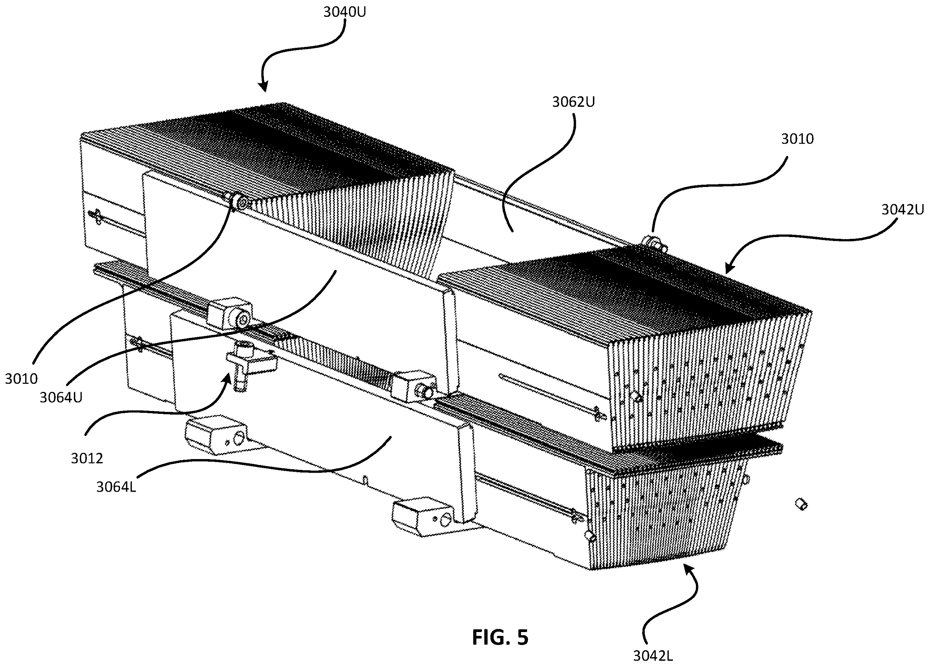

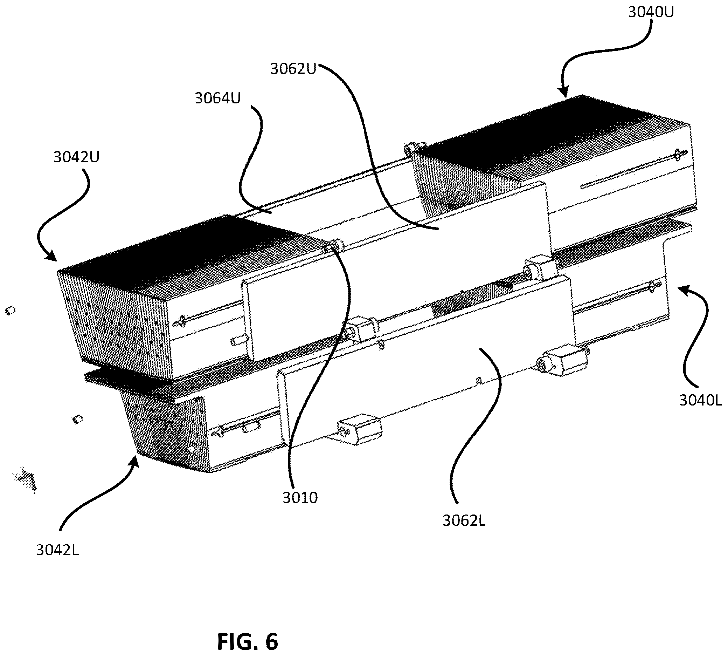

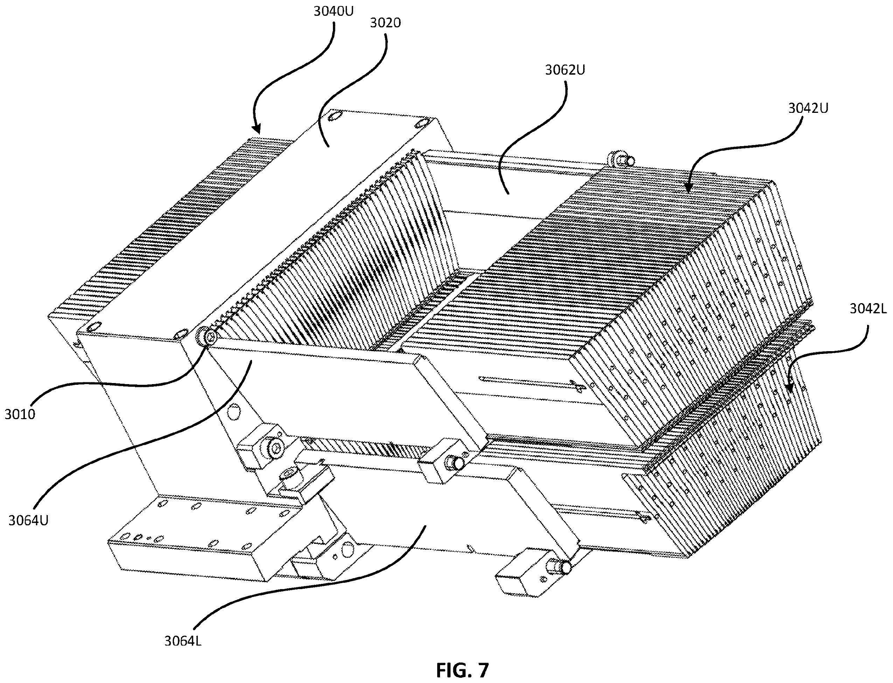

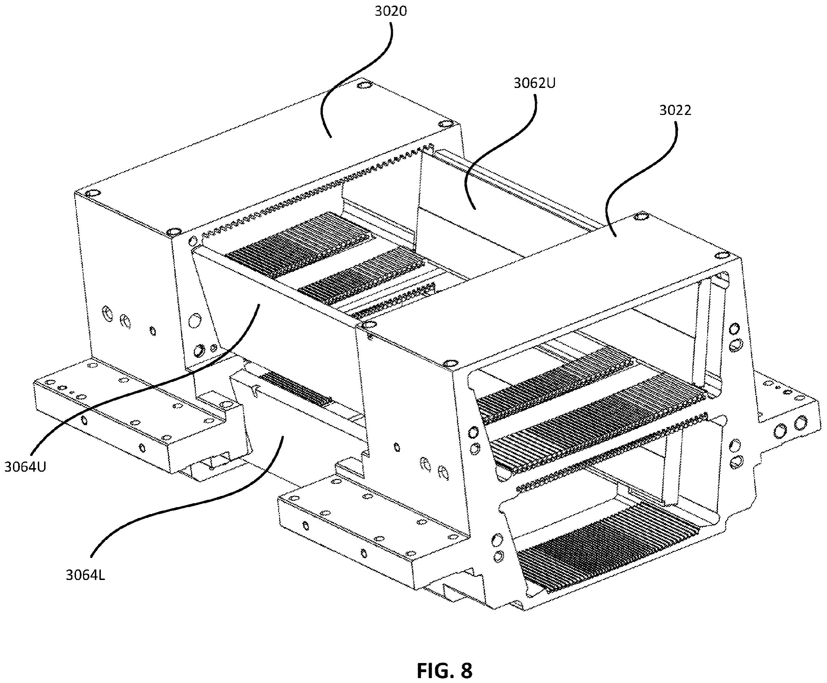

FIGS. 1, 2A, 2B and 3 illustrate various views of a MLC according to example embodiments. FIG. 4 illustrates the MLC shown in FIG. 1 with the leaf boxes removed. FIGS. 5 and 6 are perspective views of the shielding elements and retaining mechanisms of the MLC shown in FIG. 1. FIG. 7 illustrates a perspective view of a leaf box and the portion of the MLC shown in FIGS. 5 and 6. FIG. 8 is a perspective view of leaf boxes and outboard shielding elements of the MLC shown in FIG. 1.

Referring to FIGS. 1-8, the MLC 120 includes a first leaf box 3020 and a second leaf box 3022 arranged opposite one another. The first leaf box 3020 is configured to move in a first direction (X-direction). The second leaf box 3022 is also configured to move in the first direction such that the first leaf box 3020 and the second leaf box 3022 move linearly toward and away from one another. The first leaf box 3020 and the second leaf box 3022 may be moved by respective carriage motors (not shown). Because carriage motors are generally known, a detailed discussion is omitted.

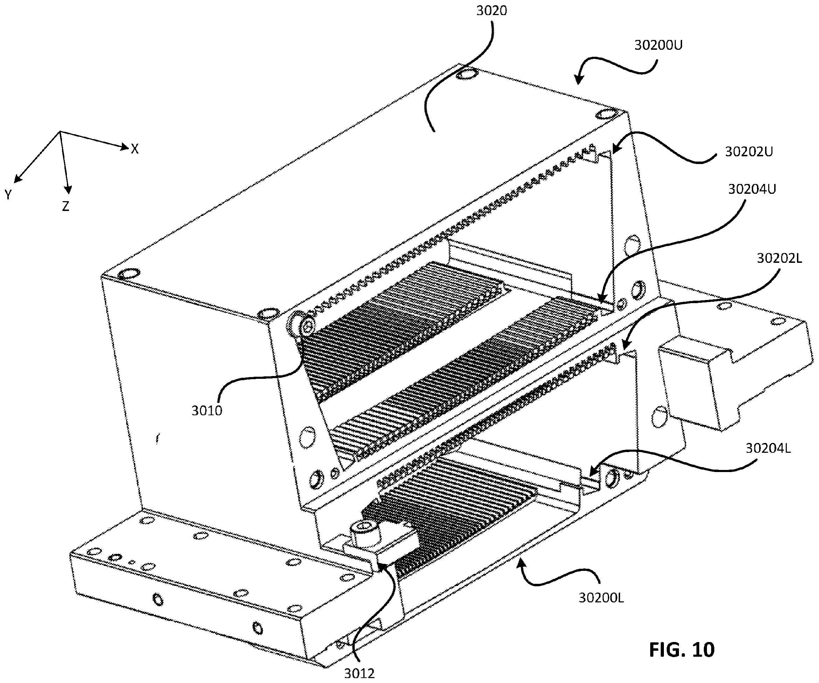

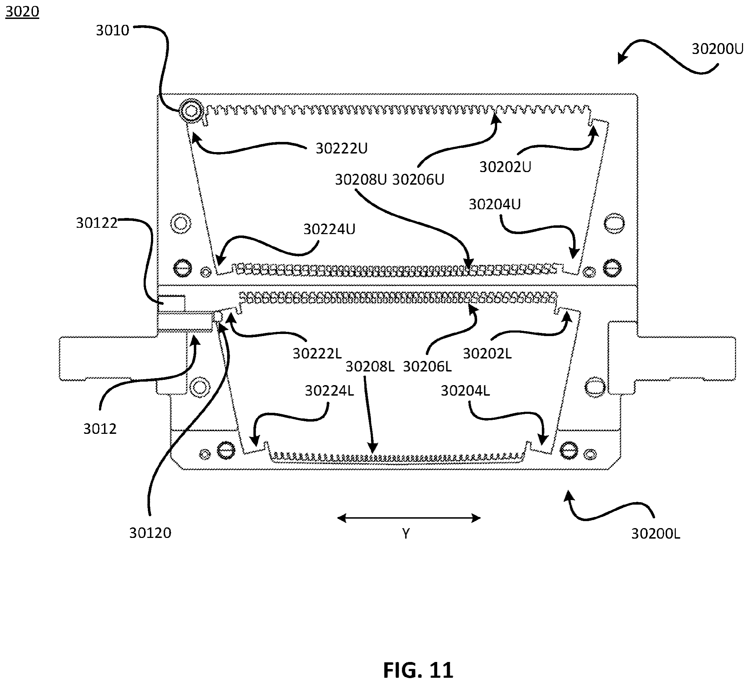

The first leaf box 3020 includes a proximal portion (30200U in FIGS. 10 and 11 discussed below) and a distal portion (30200L in FIGS. 10 and 11 discussed below). In the proximal portion, the first leaf box 3020 is configured to hold a plurality of first proximal beam blocking leaves 3040U (also referred to as upper beam blocking leaves) arranged adjacent to one another (side-by-side) in a first linear array or row. Each of the first proximal beam blocking leaves 3040U is configured to move independently in the first direction relative to one another and relative to the first leaf box 3020. Each of the plurality of first proximal beam blocking leaves 3040U may be moved/driven independently by drive assembly 3080U, which includes one or more drive motors.

In the distal portion, the first leaf box 3020 is configured to hold a plurality of first distal beam blocking leaves 3040L arranged adjacent to one another (side-by-side) in a linear array or row below the plurality of first proximal beam blocking leaves 3040U. Each of the first distal beam blocking leaves 3040L is also configured to move independently in the first direction relative to one another, relative to the first leaf box 3020 and relative to the plurality of first proximal beam blocking leaves 3040U. The plurality of first distal beam blocking leaves 3040L may be moved/driven independently by drive assembly 3080L, which includes one or more drive motors.

The second leaf box 3022 also includes a proximal portion and a distal portion. In the proximal portion, the second leaf box 3022 is configured to hold a plurality of second proximal beam blocking leaves 3042U arranged adjacent to one another (side-by-side) in a second linear array or row. Each of the second proximal beam blocking leaves 3042U is configured to move independently relative to one another and relative to the second leaf box 3022. The second proximal beam blocking leaves 3042U may also be configured to move relative to the plurality of first proximal beam blocking leaves 3040U. The plurality of second proximal beam blocking leaves 3042U may be moved/driven independently by drive assembly 3082U, which includes one or more drive motors.

In the distal portion, the second leaf box 3022 is configured to hold a plurality of second distal beam blocking leaves 3042L arranged adjacent to one another (side-by-side) in a linear array or row below the plurality of second proximal beam blocking leaves 3042U. Each of the second distal beam blocking leaves 3042L are configured to move independently relative to one another, relative to the plurality of second proximal beam blocking leaves 3042U, relative to the first and second leaf boxes 3020 and 3022, and relative to the beam blocking leaves 3040U and 3040L. The plurality of second distal beam blocking leaves 3042L may be moved/driven independently by drive assembly 3082L, which includes one or more drive motors.

According to one or more example embodiments, the first proximal beam blocking leaves 3040U and the second proximal beam blocking leaves 3042U are arranged in pairs (e.g., one first proximal beam blocking leaf and one second proximal beam blocking leaf), wherein each pair of proximal beam blocking leaves may move independently (e.g., based on a radiation therapy treatment plan) to block or allow a radiation beam to pass through to the patient.

Similarly, the first distal beam blocking leaves 3040L and the second distal beam blocking leaves 3042L are arranged in pairs (e.g., one first distal beam blocking leaf and one second distal beam blocking leaf), wherein each pair of distal beam blocking leaves may move independently (e.g., based on a radiation therapy treatment plan) to block or allow a radiation beam to pass through to the patient.

Still referring to FIGS. 1-8, the MLC 120 further includes edge defining leaves 3062U and 3064U extending between the proximal portions of the first leaf box 3020 and the second leaf box 3022.

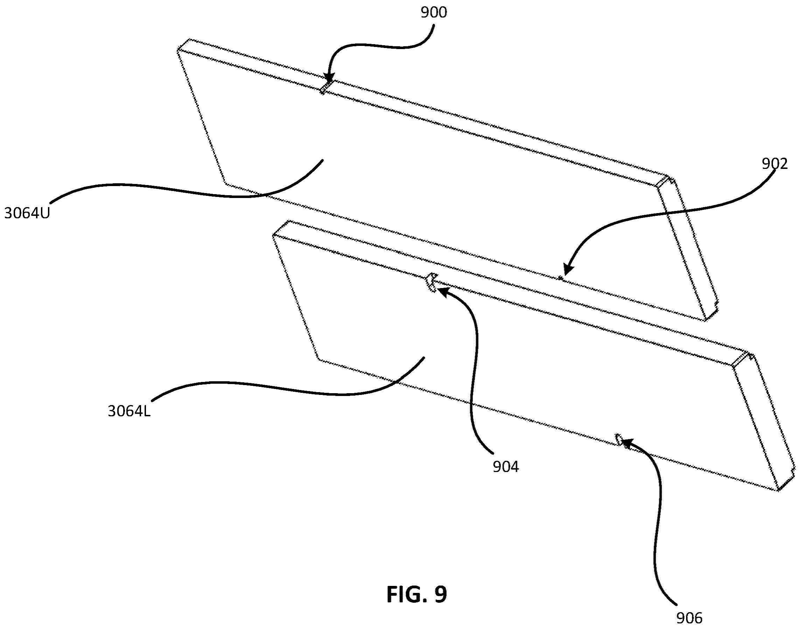

FIG. 9 illustrates an example embodiment of the edge defining leaf 3064U. As shown in FIG. 9, the edge defining leaf 3064U is a flat rectangular shaped beam blocking material having notches 900 and 902 at opposing longitudinal edges. As discussed in more detail later, the notches 900 and/or 902 allow the movement of the edge defining leaf 3064U to be fixed or stationary relative to a leaf box. The notches 900 and 902 may be formed in the material such that the beam blocking leaf may be positioned in multiple orientations. A washer and screw assembly may engage with a notch 900 or 902 and a respective leaf box such that the edge defining leaf 3064U moves along with the respective leaf box.

Returning to FIGS. 1-8, the edge defining leaves 3062U and 3064U are arranged at respective ends of the rows of proximal beam blocking leaves 3040U and 3042U to define respective edges of a radiation therapy treatment field. In the example embodiment shown in FIGS. 1-8, movement of the edge defining leaf 3064U is fixed relative to the first leaf box 3020 such that the edge defining leaf 3064U moves linearly with the first leaf box 3020 and moves relative to the second leaf box 3022 in the X-direction. For example, the edge defining leaf 3064U slides in and out of the second leaf box 3022 guided by upper and lower proximal grooves. As discussed in more detail later, the movement of the edge defining leaf 3064U may be restricted and/or fixed by a screw and washer assembly fixed to the first leaf box 3020.

Movement of the edge defining leaf 3062U is fixed relative to the second leaf box 3022 such that the edge defining leaf 3062U moves linearly with the second leaf box 3022 and moves relative to the first leaf box 3020 in the X-direction. For example, the edge defining leaf 3062U slides in and out of the first leaf box 3020 guided by upper and lower proximal grooves. As discussed in more detail later, the movement of the edge defining leaf 3062U may be restricted and/or fixed by a screw and washer assembly fixed to the second leaf box 3022.

The MLC 120 further includes scatter-blocking leaves 3062L and 3064L extending between the distal portions of the first leaf box 3020 and the second leaf box 3022.

FIG. 9 also illustrates an example embodiment of the scatter-blocking leaf 3064L. As shown in FIG. 9, the scatter-blocking leaf 3064L is a flat rectangular shaped beam blocking material having notches 904 and 906 at opposing longitudinal edges. As discussed in more detail later, the notches 904 and/or 906 allow the movement of the scatter-blocking leaf 3064L to be fixed relative to a leaf box, similar to the edge defining leaves. The notches 904 and 906 may be formed in the material such that the scatter-blocking leaf may be positioned in multiple orientations. A retaining bracket assembly may engage with a notch 904 or 906 and a respective leaf box such that the scatter-blocking leaf 3064L moves with the respective leaf box. Although not specifically described herein, it should be understood that edge defining leaf 3062U and scatter-blocking leaf 3062L may be the same or substantially the same as edge defining leaf 3064U and scatter-blocking leaf 3064L, respectively, shown in FIG. 9.

Returning to FIGS. 1-8, the scatter-blocking leaves 3062L and 3064L are arranged at respective ends of the rows of distal beam blocking leaves 3040L and 3042L.

In at least this example, movement of the scatter-blocking leaf 3064L is fixed relative to the first leaf box 3020 such that the scatter-blocking leaf 3064L moves linearly with the first leaf box 3020 and relative to the second leaf box 3022 in the X-direction. For example, the scatter-blocking leaf 3064L slides in and out of the second leaf box 3022 guided by upper and lower distal grooves.

Similarly, movement of the scatter-blocking leaf 3062L is fixed relative to the second leaf box 3022 such that the scatter-blocking leaf 3062L moves linearly with the second leaf box 3022 and relative to the first leaf box 3020 in the X-direction. For example, the scatter-blocking leaf 3062L slides in and out of the first leaf box 3020 guided by upper and lower distal grooves.

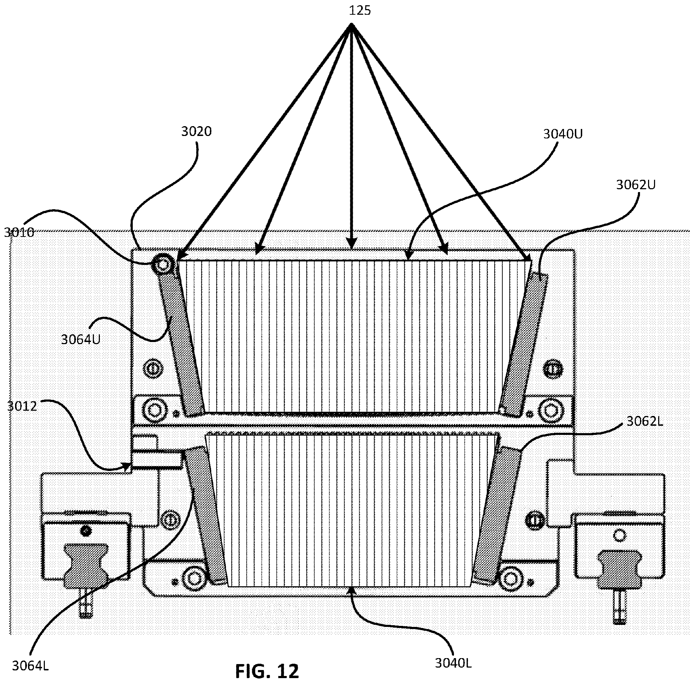

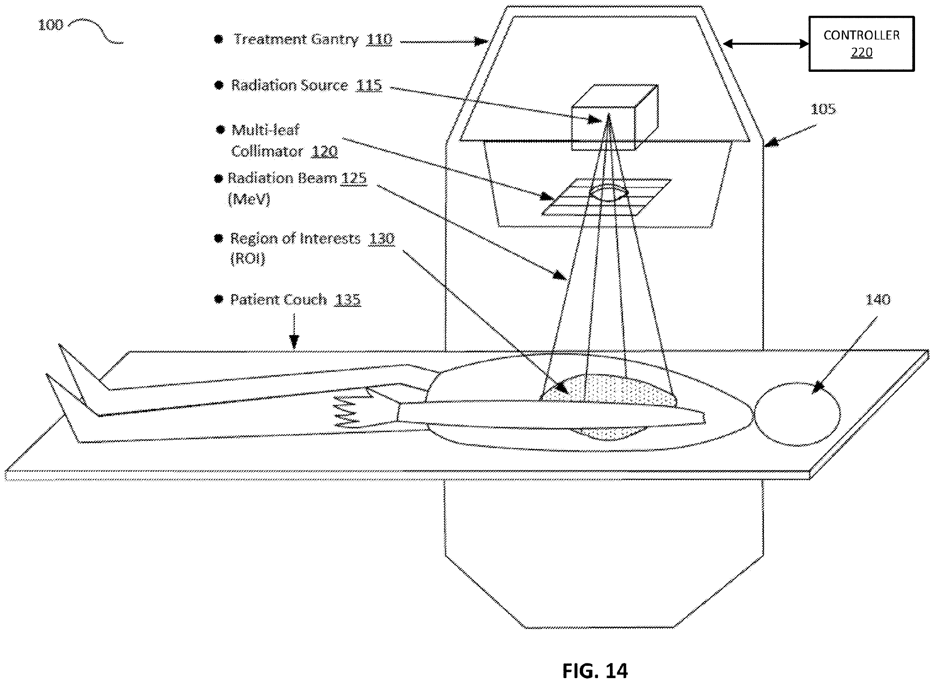

FIGS. 10 and 11 illustrate the first leaf box 3020 of FIG. 1 in more detail. FIG. 12 illustrates the first leaf box 3020 including the edge defining leaves 3062U and 3064U, the scatter-blocking leaves 3062L and 3064L, the first proximal beam blocking leaves 3040U, the first distal beam blocking leaves 3040L, and radiation beam 125 (also shown in FIG. 14). For the sake of brevity, only the first leaf box 3020 will be described. It should be understood, however, that the second leaf box 3022 may be the same or substantially the same as the first leaf box 3020. When implemented in a MLC according to one or more example embodiments, the second leaf box 3022 may be the same as the first leaf box 3020, but rotated 180 degrees about a vertical axis.

Referring to FIGS. 10-12, the first leaf box 3020 includes a proximal portion 30200U and a distal portion 30200L.

The proximal portion 30200U includes first upper proximal outer groove 30202U and first lower proximal outer groove 30204U to hold the edge defining leaf 3062U at an edge of a radiation therapy treatment field as the edge defining leaf 3062U moves relative to the first leaf box 3020. At an opposite side of the proximal portion 30200U in the Y-direction, the proximal portion 30200U includes second upper proximal outer groove 30222U and second lower proximal outer groove 30224U to hold the edge defining leaf 3064U at an edge of a radiation therapy treatment field as the edge defining leaf 3064U moves along with the first leaf box 3020.

The proximal portion 30200U of the first leaf box 3020 further includes a plurality of upper proximal inner grooves 30206U between the upper proximal outer grooves 30202U and 30222U, and a plurality of lower proximal inner grooves 30208U between the lower proximal outer grooves 30204U and 30224U.

The pluralities of upper and lower proximal inner grooves 30206U and 30208U hold and guide the plurality of first proximal beam blocking leaves 3040U during movement thereof.

The distal portion 30200L includes first upper distal outer groove 30202L and first lower distal outer groove 30204L to hold the scatter-blocking leaf 3062L in place to block scatter radiation and to guide the scatter-blocking leaf 3062L as the scatter-blocking leaf 3062L moves relative to the first leaf box 3020. At an opposite side in the Y-direction, the distal portion 30200L includes second upper distal outer groove 30222L and second lower distal outer groove 30224L to hold the scatter-blocking leaf 3064L in place to block scatter radiation as the scatter-blocking leaf 3064L moves along with the first leaf box 3020.

The distal portion 30200L of the first leaf box 3020 further includes a plurality of upper distal inner grooves 30206L between the upper distal outer grooves 30202L and 30222L, and a plurality of lower distal inner grooves 30208L between the lower distal outer grooves 30204L and 30224L.

The pluralities of upper and lower distal inner grooves 30206L and 30208L hold and guide the plurality of first distal beam blocking leaves 3040L during movement thereof.

A screw and washer assembly 3010 engages with the first leaf box 3020 above the second upper proximal outer groove 30222U. As shown in FIGS. 1-5 and 12, for example, a washer portion of the screw and washer assembly 3010 engages with the notch 900 to restrict movement of the edge defining leaf 3064U relative to the first leaf box 3020 during operation. That is, for example, the screw and washer assembly 3010 engages with the first leaf box 3020 and the notch 900 (or 902 depending on orientation) such that the edge defining leaf 3064U moves along with the first leaf box 3020. As noted above, the edge defining leaf 3064U spans or extends between the first leaf box 3020 and the second leaf box 3022. At the second leaf box 3022, the edge defining leaf 3064U is allowed to slide and move relative to the second leaf box 3022 as the first leaf box 3020 moves in the X-direction.

In one example, the screw portion of the screw and washer assembly 3010 may be a socket head cap screw.

Still referring to FIGS. 10-12, a retaining bracket assembly 3012, which is secured by a screw and washer assembly 30122, has a protruding portion 30120 that extends in the Y-direction and engages with the notch 904 to restrict movement of the scatter-blocking leaf 3064L relative to the first leaf box 3020 during operation. That is, for example, the retaining bracket assembly 3012 engages with the notch 904 (or 906 depending on orientation) such that the scatter-blocking leaf 3064L moves along with the first leaf box 3020 in the same or substantially the same manner as the edge defining leaf 3064U. Similar to the edge defining leaf 3064U, at the second leaf box 3022, the scatter-blocking leaf 3064L is allowed to slide and move relative to the second leaf box 3022 as the first leaf box 3020 is moved.

In one example, the screw portion of the screw and washer assembly 30122 may be a socket head cap screw.

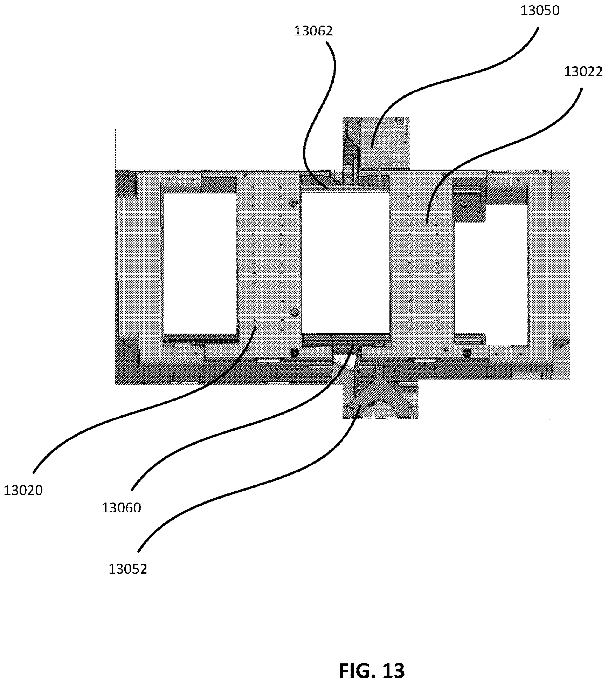

According to at least one other example embodiment, the edge defining leaves and the scatter-blocking leaves may be fixed in position by a retaining bracket or mechanism at a middle portion of the respective outboard shielding element. In this example embodiment, each leaf box moves relative to the outboard shielding elements. This example embodiment is illustrated in FIG. 13.

Because the example embodiment shown in FIG. 13 is similar to the example embodiments described above, only the differences will be described herein.

In more detail, the example embodiment shown in FIG. 13 is similar to the example embodiment shown and described with regard to FIGS. 1-12, except that each of the edge defining leaves 13060 and 13062, and the scatter-blocking leaves, are fixed in position by a respective one of retaining brackets 13050 and 13052. Unlike the example embodiments shown and described with regard to FIGS. 1-12, in this example embodiment, the respective leaf boxes 13020 and 13022 move relative to the respective outboard shielding elements.

FIG. 14 illustrates a radiation therapy treatment machine including the MLC 120, according to example embodiments.

Referring to FIG. 14, the radiation therapy treatment machine 100 includes a patient couch 135, on which a patient 140 may be positioned so that the region of interest 130 is properly located within the radiation beam 125. The treatment gantry 110 includes a radiation source 115 and the MLC 120. The radiation source 115 directs the radiation beam 125, through the MLC 120, and towards the region of interest (ROI) 130. Individual beam blocking leaves and outboard shielding elements of the MLC 120 are arranged to block portions of the radiation beam 125 that fall outside the region of interest 130 to define the radiation therapy treatment field.

In some example embodiments, the patient couch 135 includes multiple movable parts (not illustrated) to position the patient couch 135 under the treatment gantry 110 and next to, within, or partially within the treatment unit 105. Furthermore, in some example embodiments, the treatment gantry 110 may include movable parts that enable the treatment gantry 110 to be rotated about the patient couch 135 or otherwise moved relative to the region of interest 130. Movement of the treatment gantry 110 or the patient couch 135 may cause the region of interest 130 to move with respect to the radiation source 115 and the MLC 120. Changes in the relative position of the region of interest 130 may cause the shape and size of the region of interest to vary, which require individual beam blocking leaves of the MLC 120 to be moved to block different portions of the radiation beam 125 and modify the radiation therapy treatment field.

The radiation therapy treatment machine 100 is in two-way communication with a controller 220 configured to control operation of one or more components of the radiation therapy treatment machine 100 shown in FIG. 14 concurrently, simultaneously, separately, individually, etc.

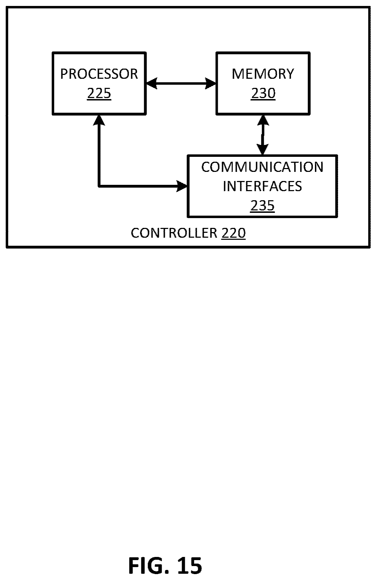

FIG. 15 is a block diagram illustrating an example embodiment of the controller 220 shown in FIG. 14.

Referring to FIG. 15, the controller 220 includes at least one processor 225, at least one memory 230, and at least one communication interface 235. The at least one memory 230 may be configured to store instructions that may be executed by the at least one processor 225 to cause the radiation therapy treatment machine 100 to perform one or more functions such as executing a radiotherapy procedure, control the MLC 120, etc.

As will be appreciated, depending on the implementation of the controller 220, the controller 220 may include additional components. However, it is not necessary that all of these generally conventional components be shown in order to illustrate example embodiments. For example purposes, the controller 220 will be discussed with regard to the processor 225. However, it should be understood that the controller 220 may include one or more processors or other processing circuitry, such as one or more Application Specific Integrated Circuits (ASICs).

The memory 230 may be a computer readable storage medium that generally includes a random access memory (RAM), read only memory (ROM), and/or a permanent mass storage device, such as a disk drive. The memory 230 may also store an operating system and any other routines/modules/applications for providing the functionalities of the controller 220 to be executed by the processor 225. These software components may also be loaded from a separate computer readable storage medium into the memory 230 using a drive mechanism (not shown). Such separate computer readable storage medium may include a disc, tape, DVD/CD-ROM drive, memory card, or other like computer readable storage medium (not shown). In some example embodiments, software components may be loaded into the memory 230 via one of the various communication interfaces 235, rather than via a computer readable storage medium.

The processor 225 or other processing circuitry may be configured to carry out instructions of a computer program by performing the arithmetical, logical, and input/output operations of the system. Instructions may be provided to the processor 225 by the memory 230.

The various communication interfaces 235 may be wired or wireless and may include components that interface the processor 225 with the other input/output components and/or one or more communications networks. As will be understood, the various communication interfaces 235 and programs stored in the memory 230 to set forth the special purpose functionalities of the controller 220 may vary depending on the implementation of MLC 120 and/or the radiation therapy treatment machine 100.

The various communication interfaces 235 may also include one or more user input devices (e.g., a keyboard, a keypad, a mouse, or the like) and user output devices (e.g., a display, a speaker, or the like).

According to a first aspect, a multi-leaf collimator to define a treatment field for a radiation therapy treatment machine is suggested, the multi-leaf collimator comprising: a first leaf box configured to move in a first direction and configured to hold a plurality of first beam blocking leaves arranged in a first row, wherein the plurality of first beam blocking leaves are configured to move relative to one another and relative to the first leaf box; a second leaf box arranged opposite the first leaf box, the second leaf box configured to move in the first direction and configured to hold a plurality of second beam blocking leaves arranged in a second row, wherein the plurality of second beam blocking leaves are configured to move relative to one another, configured to move relative to the plurality of first beam blocking leaves, and configured to move relative to the second leaf box; and at least a first edge defining leaf extending between the first leaf box and the second leaf box, the first edge defining leaf arranged at a first end of the first row and at a first end of the second row, wherein the first edge defining leaf is fixed relative to the first leaf box and configured to move relative to the second leaf box.

According to an embodiment, the first edge defining leaf defines an edge of the treatment field.

According to a further embodiment, the multi-leaf collimator further comprises a second edge defining leaf extending between the first leaf box and the second leaf box, the second edge defining leaf arranged at a second end of the first row and a second end of the second row, wherein the second edge defining leaf is fixed relative to the second leaf box and configured to move relative to the first leaf box.

According to a further embodiment, the first leaf box is configured to hold a plurality of third beam blocking leaves arranged in a third row below the plurality of first beam blocking leaves, wherein the plurality of third beam blocking leaves are configured to move relative to one another and relative to the first leaf box; the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box; and the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

According to a further embodiment, the multi-leaf collimator further comprises a second scatter-blocking leaf extending between the first leaf box and the second leaf box, the second scatter-blocking leaf arranged below the second edge defining leaf at a second end of the third row and a second end of the fourth row, wherein the second scatter-blocking leaf is fixed relative to the second leaf box and configured to move relative to the first leaf box.

According to a further embodiment, the first edge defining leaf and the second edge defining leaf at least partly define edges of the treatment field.

According to a further embodiment, the first leaf box is configured to hold a plurality of third beam blocking leaves arranged in a third row below the plurality of first beam blocking leaves, wherein the plurality of third beam blocking leaves are configured to move relative to one another and relative to the first leaf box; the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box; and the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

According to a further embodiment, the first leaf box is configured to move the first edge defining leaf relative to the second leaf box, and the second leaf box is configured to move the second edge defining leaf relative to the first leaf box.

According to a further embodiment, the first edge defining leaf, the plurality of first beam blocking leaves, and the plurality of second beam blocking leaves are configured to move relative to one another.

According to a further embodiment, the first leaf box is configured to move the first edge defining leaf relative to the second leaf box, and the second leaf box includes a guide component to hold the first edge defining leaf at an edge of the treatment field as the first edge defining leaf moves relative to the second leaf box.

According to a second aspect, a radiation therapy treatment machine is suggested, the radiation therapy treatment machine comprising: a radiation source configured to emit a radiation beam; and a multi-leaf collimator to block portions of the radiation beam to define a treatment field for the radiation therapy treatment machine, the multi-leaf collimator including a first leaf box configured to move in a first direction and configured to hold a plurality of first beam blocking leaves arranged in a first row, wherein the plurality of first beam blocking leaves are configured to move relative to one another and relative to the first leaf box, a second leaf box arranged opposite the first leaf box, the second leaf box configured to move in the first direction and configured to hold a plurality of second beam blocking leaves arranged in a second row, wherein the plurality of second beam blocking leaves are configured to move relative to one another, configured to move relative to the plurality of first beam blocking leaves, and configured to move relative to the second leaf box, and at least a first edge defining leaf extending between the first leaf box and the second leaf box, the first edge defining leaf arranged at a first end of the first row and at a first end of the second row, wherein the first edge defining leaf is fixed relative to the first leaf box and configured to move relative to the second leaf box.

According to an embodiment, the first edge defining leaf defines an edge of the treatment field.

According to a further embodiment, the multi-leaf collimator further comprises: a second edge defining leaf extending between the first leaf box and the second leaf box, the second edge defining leaf arranged at a second end of the first row and a second end of the second row, wherein the second edge defining leaf is fixed relative to the second leaf box and configured to move relative to the first leaf box.

According to a further embodiment, the first leaf box is configured to hold a plurality of third beam blocking leaves arranged in a third row below the plurality of first beam blocking leaves, wherein the plurality of third beam blocking leaves are configured to move relative to one another and relative to the first leaf box, the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box, and the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

According to a further embodiment, the multi-leaf collimator further comprises: a second scatter-blocking leaf extending between the first leaf box and the second leaf box, the second scatter-blocking leaf arranged below the second edge defining leaf at a second end of the third row and a second end of the fourth row, wherein the second scatter-blocking leaf is fixed relative to the second leaf box and configured to move relative to the first leaf box.

According to a further embodiment, the first edge defining leaf and the second edge defining leaf define edges of the treatment field.

According to a further embodiment, the first leaf box is configured to move the first edge defining leaf relative to the second leaf box, and the second leaf box is configured to move the second edge defining leaf relative to the first leaf box.

According to a further embodiment, the first leaf box is configured to hold a plurality of third beam blocking leaves arranged in a third row below the plurality of first beam blocking leaves, wherein the plurality of third beam blocking leaves are configured to move relative to one another and relative to the first leaf box; the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box; and the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

According to a further embodiment, the first edge defining leaf, the plurality of first beam blocking leaves, and the plurality of second beam blocking leaves are configured to move relative to one another.

According to a further embodiment, the first leaf box is configured to move the first edge defining leaf relative to the second leaf box, and the second leaf box includes a guide component to hold the first edge defining leaf at an edge of the treatment field as the first edge defining leaf moves relative to the second leaf box.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

## Claims

1. A multi-leaf collimator to define a treatment field for a radiation therapy treatment machine, the multi-leaf collimator comprising:
a first leaf box configured to move in a first direction and configured to hold a plurality of first beam blocking leaves arranged in a first row, wherein the plurality of first beam blocking leaves are configured to move relative to one another and relative to the first leaf box;
a second leaf box arranged opposite the first leaf box, the second leaf box configured to move in the first direction and configured to hold a plurality of second beam blocking leaves arranged in a second row, wherein the plurality of second beam blocking leaves are configured to move relative to one another, configured to move relative to the plurality of first beam blocking leaves, and configured to move relative to the second leaf box; and
at least a first edge defining leaf extending between the first leaf box and the second leaf box, the first edge defining leaf arranged at a first end of the first row and at a first end of the second row, wherein the first edge defining leaf is fixed relative to the first leaf box and configured to move relative to the second leaf box.

2. The multi-leaf collimator of claim 1, wherein the first edge defining leaf defines an edge of the treatment field.

3. The multi-leaf collimator of claim 1 or 2, further comprising:
a second edge defining leaf extending between the first leaf box and the second leaf box, the second edge defining leaf arranged at a second end of the first row and a second end of the second row, wherein the second edge defining leaf is fixed relative to the second leaf box and configured to move relative to the first leaf box.

4. The multi-leaf collimator of claim 3, wherein
the first leaf box is configured to hold a plurality of third beam blocking leaves arranged in a third row below the plurality of first beam blocking leaves, wherein the plurality of third beam blocking leaves are configured to move relative to one another and relative to the first leaf box,
the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box, and
the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

5. The multi-leaf collimator of claim 4, further comprising:
a second scatter-blocking leaf extending between the first leaf box and the second leaf box, the second scatter-blocking leaf arranged below the second edge defining leaf at a second end of the third row and a second end of the fourth row, wherein the second scatter-blocking leaf is fixed relative to the second leaf box and configured to move relative to the first leaf box.

6. The multi-leaf collimator of one of claims 3 to 5, wherein the first edge defining leaf and the second edge defining leaf at least partly define edges of the treatment field.

7. The multi-leaf collimator of one of claims 1 to 6, wherein
the first leaf box is configured to hold a plurality of third beam blocking leaves arranged in a third row below the plurality of first beam blocking leaves, wherein the plurality of third beam blocking leaves are configured to move relative to one another and relative to the first leaf box,
the second leaf box is configured to hold a plurality of fourth beam blocking leaves arranged in a fourth row below the plurality of second beam blocking leaves, wherein the plurality of fourth beam blocking leaves are configured to move relative to one another, configured to move relative to the plurality of third beam blocking leaves, and configured to move relative to the second leaf box, and
the multi-leaf collimator includes a first scatter-blocking leaf extending between the first leaf box and the second leaf box, the first scatter-blocking leaf arranged below the first edge defining leaf at a first end of the third row and at a first end of the fourth row, wherein the first scatter-blocking leaf is fixed relative to the first leaf box and configured to move relative to the second leaf box.

8. The multi-leaf collimator of one of claims 3 to 7, wherein
the first leaf box is configured to move the first edge defining leaf relative to the second leaf box, and
the second leaf box is configured to move the second edge defining leaf relative to the first leaf box.

9. The multi-leaf collimator of one of claims 1 to 8, wherein the first edge defining leaf, the plurality of first beam blocking leaves, and the plurality of second beam blocking leaves are configured to move relative to one another.

10. The multi-leaf collimator of one of claims 1 to 9, wherein
the first leaf box is configured to move the first edge defining leaf relative to the second leaf box, and
the second leaf box includes a guide component to hold the first edge defining leaf at an edge of the treatment field as the first edge defining leaf moves relative to the second leaf box.

11. A radiation therapy treatment machine comprising:
a radiation source configured to emit a radiation beam; and
a multi-leaf collimator to block portions of the radiation beam to define a treatment field for the radiation therapy treatment machine, the multi-leaf collimator being embodied according to one of claims 1 to 10.
